# EUROPÄISCHE PATENTSCHRIFT

(11) **EP 0 612 312 B1**
(45) Veröffentlichungstag und Bekanntmachung des Hinweises auf die Patenterteilung: **02.07.1997**
(21) Anmeldenummer: 92923576.0
(22) Anmeldetag: 13.11.1992
(51) Int. Cl.: C07D 209/20, C07C 271/20

(54) **SYNTHESE VON TRICYCLO [3.3.1.13,7]DEC-2-YL R-(R*,R*)-3-(1H-INDOL-3-YL-METHYL)-3-METHYL-4,9-DIOXO-7,11-DIPHENYL-10-OXA-2,5,8-TRIAZA-UNDECANAT**
SYNTHESIS OF TRICYCLO [3.3.1.13,7]DEC-2-YL R-(R*,R*)-3-(1H-INDOL-3-YL-METHYL)-3-METHYL-4,9-DIOXO-7,11-DIPHENYL-10-OXA-2,5,8-TRIAZA-UNDECANATE
SYNTHESE DU TRICYCLO [3.3.1.13,7]DEC-2-YL R-(R*,R*)-3-(1H-INDOL-3-YL-METHYL)-3-METHYL-4,9-DIOXO-7,11-DIPHENYL-10-OXA-2,5,8-TRIAZA-UNDECANATE

(30) Priorität: 14.11.1991 DE 4137490
(43) Veröffentlichungstag der Anmeldung: 31.08.1994
(73) Patentinhaber: GÖDECKE AKTIENGESELLSCHAFT, D-10587 Berlin (DE)
(72) Erfinder: HERRMANN, Wolfgang, Dr., 79104 Freiburg (DE); STEINER, Klaus, Dr., D-79312 Emmendingen (DE); WITZKE, Hans-Joachim, Dr., 79331 Nimburg (DE)
(74) Vertreter: Mansmann, Ivo
(86) Internationale Anmeldenummer: EP9202613
(87) Internationale Veröffentlichungsnummer: WO9310090

(56) Entgegenhaltungen:
- EP-A- 0 290 888
- WO-A-91/00274
- Beilsteins Handbuch der Organischen Chemie, Bd. XII, 1929, Springer-Verlag, Berlin
- J. MED. CHEM., Bd. 34, Nr. 1, 1991, Seiten 404-414; D.C. HORWELL et al.: "Rationally designed dipeptoid analogs of CCK. Alpha-methyltryptophan derivatives as highly selective and orally active gastrin and CCK-B antagonists with potent anxiolytic properties"
- WORLD PATENTS INDEX, Woche 7440, Derwent Publications Ltd., London, GB, AN 74-70504V & JP A 49 033 958
- WORLD PATENTS INDEX LATEST, Woche 9032, Derwent Publications Ltd., London, GB, AN 90-244529 & Research Disclosure Nr. 315091 (20-06-90)

## Beschreibung

Die Erfindung betrifft die Herstellung von Tricyclo[3.3.1.13,7]dec-2-yl[R-(R*,R*)]-3-(1H-indol-3-yl-methyl)-3-methyl-4,9-dioxo-7,11-diphenyl-10-oxa-2,5,8-triaza-undecanat der Formel I.

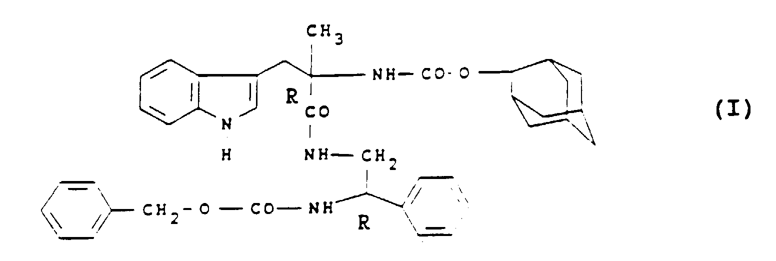

Bei dieser Verbindung handelt es sich um eine Schlüsselverbindung zur Darstellung einer neuen Klasse von hochselektiven und oral wirksamen Gastrin- und CCK-B-Antagonisten (D.C.Horwell et al, J.Med.Chem. 1991, 34, 404-414).

Aufgabe der Erfindung ist es daher, ein ökonomisches und auch technisch durchführbares Verfahren zu entwickeln um diese Schlüsselverbindung (I) in der geforderten Reinheit zu gewinnen.

Sie wird nach Schema 1 mittels der sogenannten Carbodiimid-Methode, mit 1-Hydroxybenztriazol-hydrat als Katalysator, ausgehend von einem optisch aktiven, monogeschütztem Diamin der Formel II mit dem ebenfalls optisch aktiven R-α-Methyltryptophansäure-derivat der Formel III hergestellt. Das monogeschützte Diamin (II) wird ausgehend von R-(-)-α-Phenylglycinol über N-[(Benzyloxy) carbonyls-(R) -β-amino-2-phenylethanol (IV) und N-[(Benzyloxy)carbonyl]-O-(toluene-4-sulfonyl)-(R)-β-amino-2-phenylethanol (V) hergestellt. Die letztgenannte Tosylverbindung (V) wird mit Natriumazid in DMF zum N-[(Benzyloxy)carbonyl]-(R)-β-amino-1-azido-2-phenylethan (VI) umgesetzt das anschließend zu dem monogeschützten Diamin N^{β}-[(Benzyloxy)carbonyl]-(R)-β-amino-2-phenylethan-amin (II) hydriert wird.

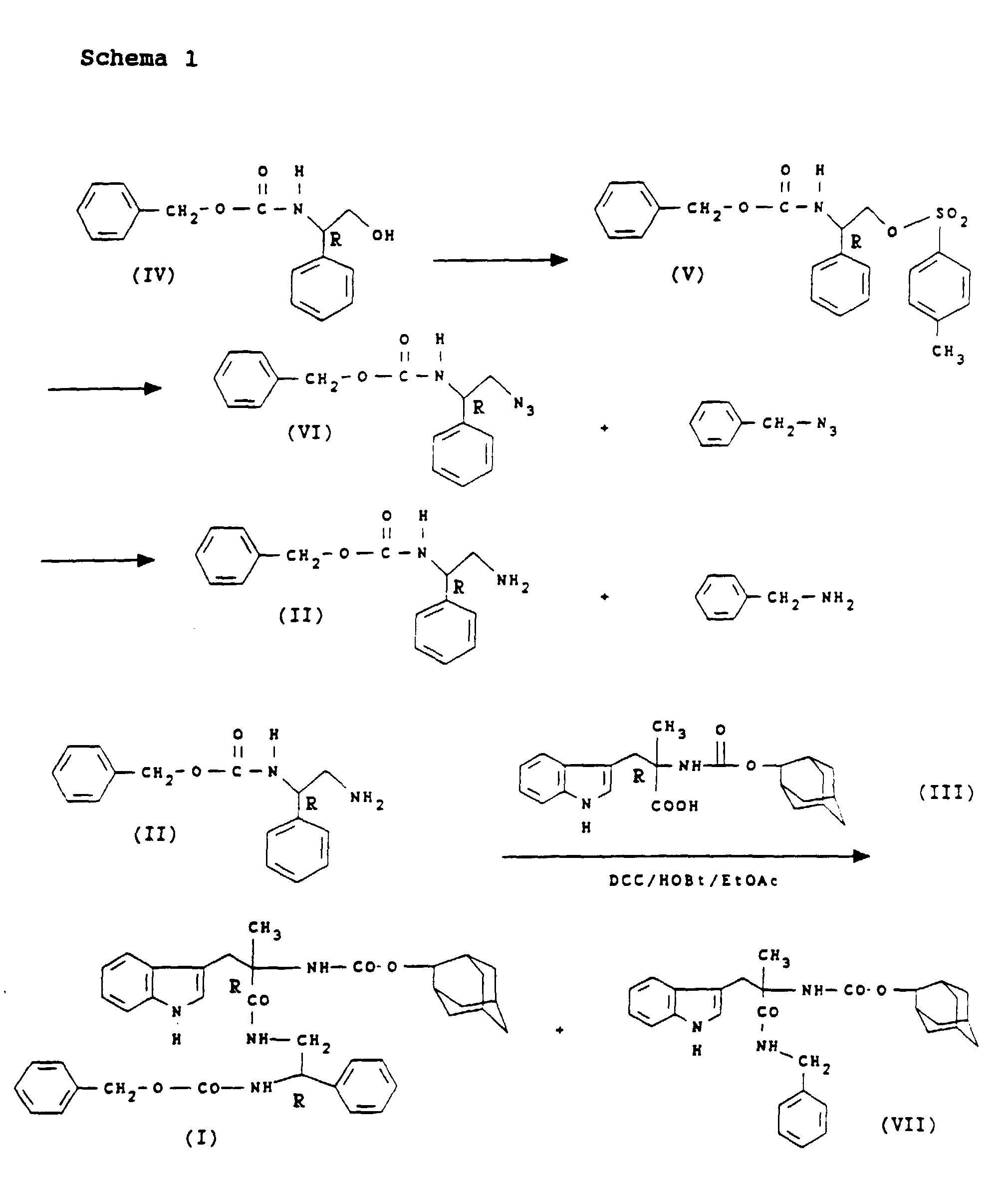

Bei dem bisher bekannten Verfahren (D.C.Horwell et al., J.Med.Chem. 1991, 34, 404-414) treten die folgenden Probleme auf. Zunächst entsteht bei der Umsetzung der Tosylverbindung der Formel V mit Natriumazid ein Rohazid, das neben der gewünschten Verbindung der Formel II noch bis zu 5 % an Benzylazid enthält, das aber auf dieser Stufe wegen der potentiellen Instabilität von Aziden nicht entfernt werden kann. Demzufolge entsteht bei der nachfolgenden Hydrierung des Azidgemisches ein Rohamingemisch, das neben dem gewünschten Amin der Formel II unter anderem auch Benzylamin enthält. Dieses Benzylamin kann ohne großen Arbeitsaufwand nicht abgetrennt werden und reagiert bei der anschließenden Kupplung mit dem R-α-Methyltryptophansäure-derivat der Formel III in analoger Weise wie das Amin II. Die hierdurch entstehende Verunreinigung der Formel VII kann nur durch aufwendige, oft mehrfache säulenchromatographische Reinigung abgetrennt werden. Da das N-β-[(Benzyloxy)carbonyl]-(R)-β-amino-2-phenylethanamin der Formel II nach diesem Verfahren nur als ein öliges Rohgemisch anfällt, dessen genauer Gehalt am gewünschten Produkt nur schwer feststellbar ist, wird es für die anschließende Kupplungsreaktion in einem ca. 45 %igen überschuß eingesetzt. Das hierbei entstehende Reaktionsgemisch muß mittels einer äusserst aufwendigen und zeitraubenden säulenchromatographischen Trennung gereinigt werden, um ein für weitere Umsetzungen genügend reines Endprodukt (I) zu erhalten. Weiterhin erwies sich das N-β-[(Benzyloxy)carbonyl]-(R)-β-amino-2-phenylethanamin (II) als nicht lagerstabil, da dieses Amin CO₂ aus der Luft absorbiert und hierbei teilweise in ein Carbonat übergeht, das in den für die Kupplung verwendeten Lösungsmitteln unlöslich ist.

Überraschenderweise wurde gefunden, daß das Amin (II) ein beständiges, schwerlösliches, nichthygroskopisches, stöchiometrisches Salz (VIII) mit Kohlensäure bildet, wenn das Rohazid ohne jede weitere Reinigung in Essigester in Gegenwart eines Katalysators, wie z.B. von Raney-Nickel, hydriert wird, zur klarfiltrierten Lösung geringe Mengen Alkohol zugefügt werden und anschließend gasförmiges CO₂ eingeleitet oder festes CO₂ zugegeben wird nach dem Schema 2, 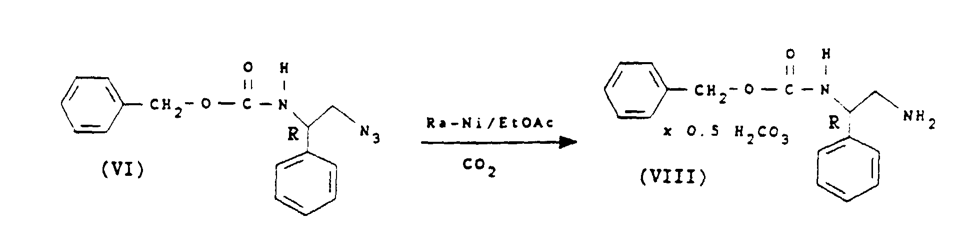 wobei das Carbonat (VIII) selektiv nahezu quantitativ mit einer Reinheit > 98 abs% ausgefällt wird. Benzylamin bleibt als Verunreinigung in der Mutterlauge zurück.

Weiterhin wurde überraschenderweise gefunden, daß sich das Carbonatsalz des Amins (VIII) in stöchiometrischer Menge direkt, d.h. ohne Freisetzen des Amins (II), mit dem R-α-Methyltryptophansäure-derivat (III) zur Titelverbindung (I) umsetzen läßt gemäß Schema 3:

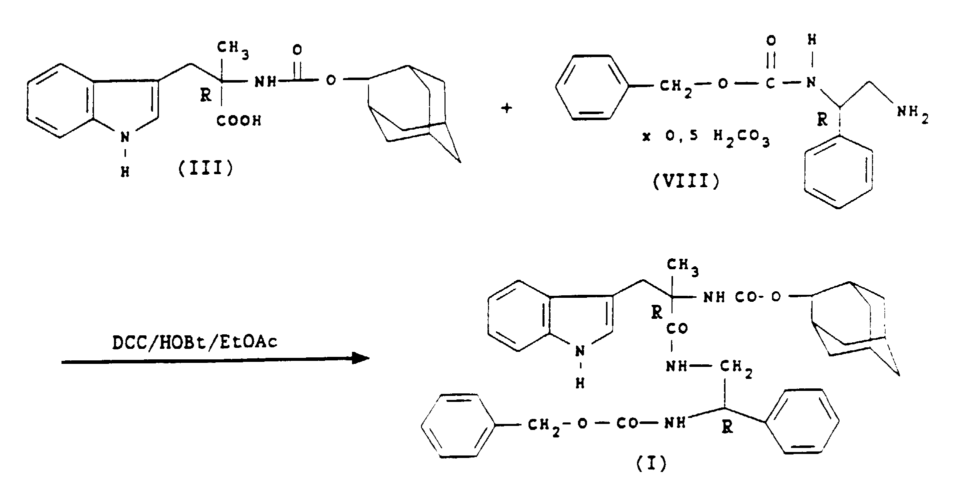

Bei diesem Verfahren fällt die Titelverbindung (I) in nahezu quantitativer Ausbeute und ohne säulenchromatographische Reinigung in einer Reinheit von ca. 98 % an und kann direkt für die weitere Synthese verschiedener Wirkstoffe eingesetzt werden.

Die erfindungsgemäßen Verfahren werden an den folgenden Beispielen näher erläutert:

### Beispiel 1:

### N^{β}-[(Benzyloxy)carbonyl]-(R)-β-amino-2-phenylethanamincarbonat

22,7 g N-[(Benzyloxy)carbonyl]-(R)-β-amino-1-azido-2-phenylethan wurden in 300 ml Essigester gelöst und in Gegenwart von 7,3 g Raney-Nickel (B 113 W, Degussa) während 15 Stunden bei 25°C und 80 atm hydriert. Die Hydrierlösung wurde klarfiltriert und anschließend mit 90 ml Ethanol versetzt. In diese Lösung wurde gasförmiges CO₂ eingeleitet. Hierbei fiel ein weißes Produkt aus. Der Nutschkuchen wurde mit wenig Essigester/Ethanol (10:3) gewaschen. Das Produkt wurde im Umlufttrockenschrank bei 40°C getrocknet. Die Ausbeute an N^{β}-[(Benzyloxy)carbonyl]-(R)-β-amino-2-phenylethanamin-carbonat betrug 18,5 g = 79,5%; Fp.: 136,3°C; [a]_{D} -34,1° (c = 1/MeOH)).

### Beispiel 2:

### Tricyclo[3.3.1.13,7]dec-2-yl[R-(R∗,R∗)]-3-(1H-indol-3-yl-methyl)-3-methyl-4,9-dioxo-7,11-diphenyl-10-oxa-2,5,8-triaza-undecanat

4,08 g N-[(2-Adamantyloxy)carbonyl]-α-methyl-R-tryptophan wurden in 25 ml Essigester gelöst und bei 20°C mit 1,53 g 1-Hydroxy-1H-benztriazol-hydrat und 2,06 g Dicyclohexylcarbodiimid versetzt. Nach zweistündigem Rühren bei 20°C wurde von dem ausgefallenen Dicyclohexylharnstoff (2,12 g = 94,6%) abfiltriert. Zu dem klaren Filtrat wurden während ca. 15 Min. unter Rühren 3,01 g N^{β}-[(Benzyloxy)carbonyl]-(R)-β-amino-2-phenylethanamin-carbonat zugegeben. Unter CO₂-Bildung ging das Carbonat in Lösung. Das Reaktionsgemisch wurde 16 Stunden gerührt. Nach dem Klarfiltrieren wurden 25 ml Essigester zugegeben. Die Lösung wurde dreimal mit jeweils 150 ml 5 %iger Zitronensäure, dreimal mit jeweils 150 ml 5 %iger NaHCO3-Lösung und abschließend mit 25 ml Wasser gewaschen, wobei die sich eventuell bildende Emulsion durch Zugabe von NaCl gebrochen werden kann. Die organische Phase wurde über Na₂SO₄ getrocknet und filtriert. Nach dem Einengen am Rotationsverdampfer blieb ein schaumiges Produkt zurück (6,05 g = 93%; HPLC: 98,89 rel%).

## Patentansprüche

1. Verfahren zur Herstellung von Tricyclo[3.3,1.13,7]dec-2-yl[R-(R*,R*)]-3-(1H-indol-3-yl-methyl)-3-methyl-4,9-dioxo-7,11-diphenyl-10-oxa-2,5,8-triaza-undecanat, dadurch gekennzeichnet, daß N-[(Benzyloxy)carbonyl]-(R)-β-amino-1-azido-2-phenylethan der Formel VI 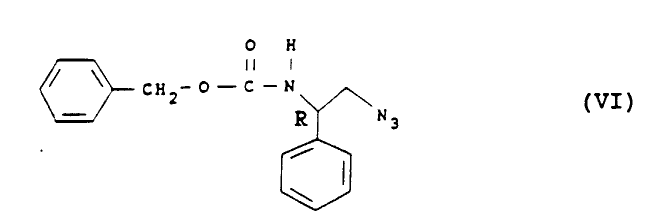 hydriert, anschließend mittels CO₂ N^{β}-[(Benzyloxy)carbonyl]-(R)-β-amino-2-phenylethanamincarbonat der Formel VIII 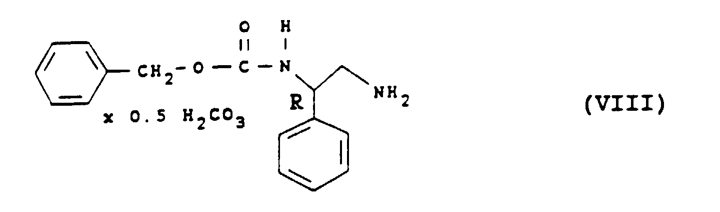 ausgefällt und dieses nach dem Carbodiimid-Verfahren mit N-[(2-Adamantyloxy)carbonyl]-α-methyl-R-tryptophan der Formel III 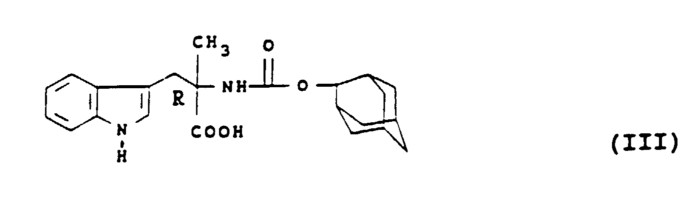 gekoppelt wird.

2. N^{β}-[(Benzyloxy)carbonyl]-(R)-β-amino-2-phenylethanamincarbonat.

## Claims

1. Process for preparing tricycle[3,3,1,13,7]dec-2-yl[R-(R*,R*)]-3-(1H-indol-3-yl-methyl)-3-methyl-4,9-dioxo-7,11-diphenyl-10-oxa-2,5,8-triaza-undecanate, characterised in that N-[(benzyloxy)carbonyl]-(R)-β-amino-1-azido-2-phenylethane of the formula VI 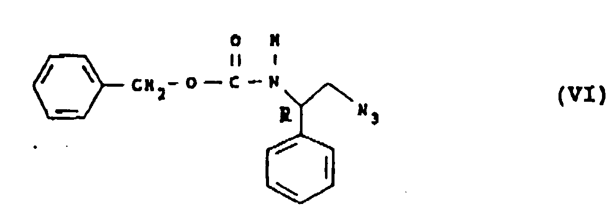 is hydrogenated, then by means of CO₂ N^{β}-[(benzyloxy)carbonyl]-(R)-β-amino-2-phenylethane amine carbonate of the formula VIII 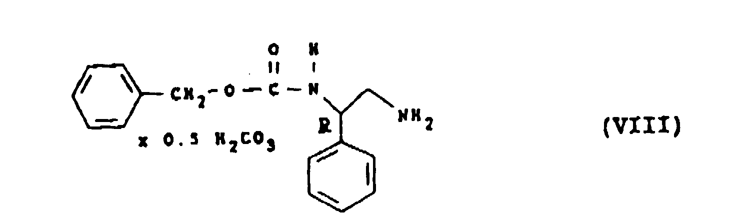 is precipitated and the latter is coupled with N-[(2-adamantyloxy)carbonyl]-α-methyl-R-tryptophan of the formula III 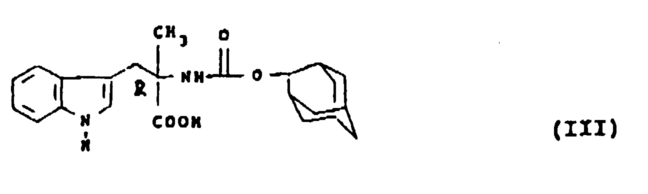 by the carbodiimide process.

2. N^{β}-[(benzyloxy)carbonyl]-(R)-β-amino-2-phenylethane amine carbonate.

## Revendications

1. Procédé de préparation du tricycle[3.3.1.13,7]dec-2-yl[R(R*,R*)]-3-(1H-indol-3-yl-méthyl)-3-méthyl-4,9-dioxo-7,11-diphényl-10-oxa-2,5,8-triaza-undecanate caractérisé en ce que l'on hydrate le N-[(benzyloxy)carbonyl]-R)-β-amino-1-azido-2-phényléthane de formule VI 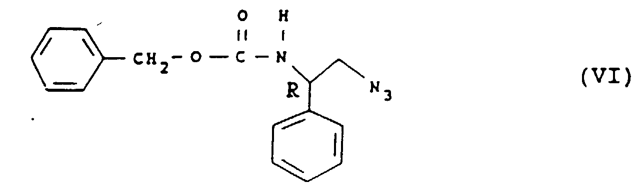 ensuite fait précipiter par action de CO₂, le N^{β}[(benzyloxy)carbonyl]-(R)-β-amino-2-phényléthanamine-carbonate de formule VIII 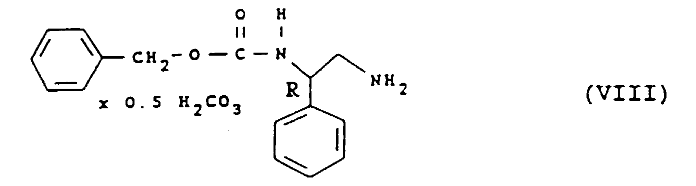 et en ce que celui-ci est couplé, selon le procédé de carbodiimidation, avec le N-[(2-adamantyloxy)carbonyl]-α-méthyl-R-tryptophan de formule III

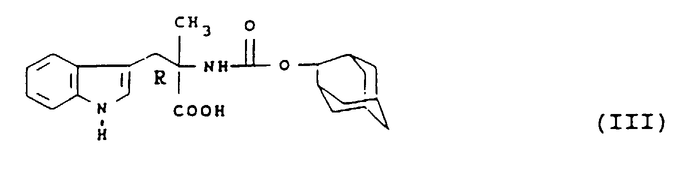

2. N^{β}-[(benzyloxy)carbonyl]-(R)-β-amino-2-phényléthanamine-carbonate.
